(19) Europäisches Patentamt
European Patent Office
Office européen des brevets

(11) Numéro de publication: 0 172 091
B1

(12) # FASCICULE DE BREVET EUROPÉEN

(45) Date de publication du fascicule du brevet: **20.05.87**

(51) Int. Cl.⁴: **C 07 C 29/136**, C 07 C 31/08, C 07 C 31/04

(21) Numéro de dépôt: **85401534.4**

(22) Date de dépôt: **25.07.85**

(54) **Procédé de production d'alcools par hydrogénolyse d'esters d'acides carboxyliques en présence d'un catalyseur contenant du nickel et de l'étain, du germanium ou du plomp.**

(30) Priorité: **08.08.84 FR 8412635**

(43) Date de publication de la demande: **19.02.86 Bulletin 86/8**

(45) Mention de la délivrance du brevet: **20.05.87 Bulletin 87/21**

(84) Etats contractants désignés: **CH DE GB IT LI**

(56) Documents cité:
**EP-A-0 095 408**
**DE-A-3 019 582**

(73) Titulaire: **INSTITUT FRANCAIS DU PETROLE, 4, Avenue de Bois- Préau, F-92502 Rueil- Malmaison (FR)**

(72) Inventeur: **Bournonville, Jean- Paul, 43, rue des Groues Vauréal, F-95000 Cergy Pontoise (FR)**
Inventeur: **Candy, Jean- Pierre, 4, chemin de la Mascotte, F-69300 Caluire (FR)**
Inventeur: **Mabilon, Gil, 36, rue de Tourville, F-78100 Saint Germain en Laye (FR)**

EP 0 172 091 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

L'invention concerne un procédé catalytique de production d'alcools par hydrogénolyse d'esters d'acides carboxyliques.

La production d'alcools, et en particulier d'alcools gras, présente un intérêt considérable pour l'industrie.

L'hydrogénolyse catalytique des esters d'acides carboxyliques représente une voie intéressante de production de ces alcools mais elle est trouvée freinée jusqu'à présent par les inconvénients des catalyseurs connus:

- les catalyseurs à base d'oxydes mixtes de cuivre et de chrome, dopés ou non, nécessitent de travailler sous pression élevée, dans la quasi totalité des cas supérieure à 20 MPa, et à une température comprise entre 250 et 350°C,

- les catalyseurs à base de métaux de transition déposés sur un support obligent en général à travailler à une température moins élevée, inférieure à 250°C et de préférence à 200°C, pour limiter la dégradation de l'alcool formé en hydrocarbures; ce qui impose des pressions opératoires en général superieures à 10 MPa pour obtenir de bonnes sélectivités à un niveau de conversion acceptable.

Plus récemment on a proposé, dans la demande de brevet européen EP-A-95 408, un catalyseur formé par une association de rhodium avec, par exemple de l'étain, déposé sur un support comme par exemple la silice ou l'alumine. Ce catalyseur permet d'obtenir dans des conditions opératoires relativement douces (température inférieure à environ 280°C et pression inférieure à environ 8 MPa) de bons rendements en alcools. Néanmoins, le coût élevé et la disponibilité limitée du rhodium sont des inconvénients importants dont il faut tenir compte et qui ont conduit la demanderesse à poursuivre des recherches dans le but de trouver un catalyseur fournissant de bons rendements en alcools, permettant de travailler dans des conditions relativement douces et ne présentant pas les inconvénients liés à l'emploi du rhodium.

Il a été découvert,de façon surprenante, qu'il est possible de réaliser l'hydrogénation d'un ester en alcool en présence d'un catalyseur contenant du nickel et au moins un second élément choisi dans le groupe constitué de l'étain, du germanium et du plomb, résultant de l'incorporation d'au moins un composé d'au moins un métal du groupe Sn, Ge et Pb à un support contenant du nickel ou à du nickel de Raney mais en aucun cas à l'alliage nickel-aluminium servant à former le nickel de Raney. Les esters d'acides carboxyliques qui peuvent être transformés en alcools sont avantageusement choisis dans le groupe des esters d'acides monocarboxyliques par exemple les esters d'acides acétique, propionique, butyrique, valérique caproïque, oléïque ou palmitique, ou polycarboxyliques en particulier dicarboxyliques comme par exemple les esters des acides oxalique, malonique ou adipique, avec des alcools alkyliques linéaires ou branchés comme par exemple le méthanol ou l'éthanol, ou des alcools aralkyliques tels que par exemple l'alcool benzylique, ou des composés hydroxyles aromatiques par exemple le phénol. Il est également possible d'hydrogéner des esters cycliques, en particulier les lactones par exemple la valérolactone ou la caprolactone. L'hydrogénation d'un ester d'un monoacide carboxylique avec un monoalcool, sans modification de la structure de la chaine hydrocarbonée, est représentée par le schéma suivant

$$R_1-\overset{\displaystyle O}{\overset{\|}{C}}-O-R_2 + 2H_2 \longrightarrow R_1-CH_2OH + R_2OH$$

où $R_1$ représente un radical hydrocarbyl, saturé ou insaturé, de 1 à 30 atomes de carbone, par exemple un radical alkyl de $C_1$ à $C_{20}$, un radical aryl de $C_6$ à $C_{22}$ ou un radical aralkyl de $C_7$ à $C_{30}$ et $R_2$ représente un radical hydrocarbyl de $C_1$ à $C_{30}$, par exemple un radical aryl de $C_6$ à $C_{22}$, un radical aralkyl de $C_7$ à $C_{30}$, ou un radical alkyl de $C_1$ à $C_{20}$, et de préférence $R_2$ représente un radical alkyl de $C_1$ à $C_{10}$ et d'une façon particulièrement préférée méthyl ou éthyl.

On opère de préférence dans un réacteur continu ou discontinu sous une pression totale comprise entre 10 et 100 bars (1 et 10 mégapascals) et de préférence entre 30 et 80 bars (3 et 8 MPa), bien que l'on puisse opérer sans inconvénient; par exemple, jusqu'à 300 bars, à une température comprise entre 180 et 330°C et de préférence entre 200 et 280°C et avec un rapport molaire hydrogène sur ester compris, par exemple, entre 2 : 1 et 50 : 1 et plus avantageusement entre 2 : 1 et 5 : 1, en présence d'un catalyseur métallique supporté renfermant les éléments suivants: le nickel dont le pourcentage pondéral est choisi entre 0,1 et 60 % et de préférence entre 2 et 10 % et au moins un élément choisi dans le groupe constitué par le germanium, l'étain et le plomb et dont le pourcentage pondéral est compris entre 0,1 et 20 % et plus particulièrement entre 1,5 et 12 %. On peut avantageusement utiliser à la fois deux des métaux du groupe ci-dessus ou même les trois métaux de ce groupe; le support, quand il en est utilisé un, peut être choisi dans le groupe constitué par la silice, les différents types d'alumine, les silices alumines, les aluminates des éléments des groupes $I_A$, $II_A$ ou $II_B$ de la classification périodique des éléments comme par exemple les aluminates de Ca, Mg, Ba, Zn, Na, K, Cd et les aluminates mixtes, et le charbon, et de préférence dans le groupe constitué par la silice et les aluminates de métaux alcalins et/ou alcalino-terreux et/ou de zinc et/ou de cadmium et les aluminates mixtes.

Il est particulièrement avantageux d'utiliser des catalyseurs dans lesquels le rapport atomique du ou des éléments du groupe Sn, Ge et Pb présents dans le catalyseur au nickel est de 0,01:1 à 4:1 et de préférence 0,1:1 à 1:1.

Le catalyseur peut être préparé par différentes procédures d'imprégnation du support et l'invention n'est pas limitée à une procédure déterminée. L'opération d'imprégnation consiste, par exemple, à mettre en contact le support préformé et une solution aqueuse ou organique d'un composé du métal ou des métaux choisi(s), le volume de solution étant de préférence en excés par rapport au volume de rétention du support ou égal à ce volume. On peut introduire le nickel et le métal additionel simultanément ou successivement. Après avoir laissé le contact entre le support et la solution pendant plusieurs heures, le support imprégné est filtré, lavé à l'eau distillée, séché et calciné sous air entre 110 et 600°C et de préférence entre 110 et 500°C. Avant utilisation, on réduit le catalyseur sous hydrogène entre 200 et 600°C et de préférence entre 300 et 500°C, cette réduction pouvant être effectuée aussitôt après calcination, ou plus tard, chez l'utilisateur.

L'élément choisi dans le groupe constitué par l'étain, le germanium et le plomb peut être introduit en solution aqueuse ou en solution hydrocarbonée selon la nature du précurseur utilisé.

D'une manière préférée le catalyseur est obtenu par imprégnation du support à l'aide d'une solution aqueuse ou organique d'au moins un composé de nickel, le volume de solution étant de préférence en excès par rapport au volume de rétention du support ou égal à ce volume. Le support imprégné est ensuite filtré, éventuellement lavé à l'eau distillée puis séché et calciné sous air entre environ 110°C et environ 600°C, et de préférence entre environ 110°C et environ 500°C, puis ensuite réduit sous hydrogène à une température comprise entre environ 200°C et environ 600°C et de préférence entre environ 300°C et environ 500°C; le produit obtenu est alors imprégné par une solution aqueuse ou organique d'un composé de germanium, d'étain et/ou de plomb; d'une manière particulièrement avantageuse on utilise une solution d'au moins un hydrocarbyl-germanium, un hydrocarbyl-étain ou un hydrocarbyl-plomb dans un hydrocarbure saturé.

Après avoir laissé le contact entre le support imprégné de nickel et la solution contenant au moins un composé de germanium, étain ou plomb pendant plusieurs heures, le produit est filtré, éventuellement lavé à l'aide du solvant employé pour déposer le germanium l'étain et/ou le plomb, séché et éventuellement calciné sous air entre environ 110°C et environ 600°C, de préférence entre environ 100°C et 500°C. Avant utilisation on réduit le catalyseur sous hydrogène entre environ 200°C et environ 600°C et de préférence entre environ 300°C et environ 500°C, cette réduction pouvant être effectuée aussitôt après la calcination, ou plus tard chez l'utilisateur.

Une autre méthode consiste à malaxer la poudre humide de support avec les précurseurs du catalyseur et à mettre ensiute en forme et sécher.

Les exemples des précurseurs métalliques utilisables dans la préparation du catalyseur sont les suivants:

Pour le nickel, on peut employer des composés tels que les chlorure, nitrate ou sels d'acides organiques solubles dans le solvant d'imprégnation, par exemple le chlorure de nickel, le nitrate de nickel, les sels d'acides organiques comme l'acétate de nickel, mais également les chlorure ou nitrate de nickel hexammine. On peut encore utiliser des composés organométalliques de nickel en solution dans un hydrocarbure, par exemple dans un hydrocarbure paraffinique saturé contenant de 5 à 12 atomes de carbone, dans un hydrocarbure naphténique contenant de 6 à 12 atomes de carbone ou dans un hydrocarbure aromatique contenant de 6 à 11 atomes de carbone; on utilisera par exemple, le dérivé avec la diméthylglyoxime. Le stéarate de nickel, le nickel dicyclopentadienyle ou le nickel dicyclopentadiene et de préférence l'acétylacétonate de nickel.

L'élément choisi dans le groupe constitué de l'étain, du germanium et du plomb peut être introduit par l'intermédiaire de composés tels que les chlorures, les bromures et le nitrate d'étain, les halogénures, nitrate et acétate de plomb, le chlorure et l'oxalate de germanium en solution aqueuse ou organique ou, de préférence sous forme d'hydrocarbyl métaux tels que des alkyls ou des arylmétaux d'étain, de germanium et de plomb, par exemple: le tétraéthyl-étain, le tétraméthyl-étain, le tétrapropyl-germanium, le tétraéthyl-plomb, le diphényl-étain, le diphényl-germanium, le tétraphényl-plomb avantageusement en solution hydrocarbonée.

Le support peut être de nature variée, comme déjà mentionné plus haut. Un support particulièrement adapté possède des caractéristiques spécifiques telles qu'une surface spécifique, déterminée par la méthode B.E.T., comprise entre 10 et 500 mètres carrés par gramme et de préference comprise entre 50 et 500 mètres carrés par gramme et un volume poreux total de 0,2 à 1,3 $cm^3/g$ de support et de préférence de 0,5 à 1,1 $cm^3/g$ de support.

Une fois les deux métaux fixés sur le support, le catalyseur subit avantageusement un traitement d'activation sous hydrogène à haute température, par exemple 300-500°C, afin d'obtenir une phase métallique active. La procédure de ce traitement sous hydrogène consiste par exemple en une montée lente de la température sous courant d'hydrogène jusqu'à la température maximale de réduction, comprise par exemple entre 300 et 500°C et de préférence entre 350 et 450°C, suivie d'un maintien pendant 1 à heures a cette température.

Un catalyseur métallique actif est également obtenu à partir d'un produit du type nickel de Raney, c'est à dire qu'il consiste au moins en partie en nickel de Raney. Ce sera par exemple un produit contenant un métal déshydrogénant traditionnel, appartenant généralement au groupe VIII de la classification périodique des éléments, par exemple le cobalt associé à du nickel de Raney, auquel on ajoute au moins un composé d'un métal ou au moins un métal du groupe formé par le germanium, l'étain et le plomb.

L'ajout d'au moins un métal additionnel du groupe Ge, Sn, Pb provoque une diminution sensible des réactions parasites, en particulier une diminution de la formation d'hydrocarbures.

L'introduction d'au moins un métal additionnel est avantageusement effectuée par injection dou des métaux additionnels dans la zone de réaction à la température réactionnelle, le précurseur du catalyseur contenant du nickel de Raney étant en suspension dans la phase liquide réactionnelle. L'injection du métal additionnel dans le milieu reactionnel est avantageusement effectuée à l'aide d'une solution d'au moins un composé

organométallique d'au moins l'un des métaux du groupe étain, plomb et germanium. Le ou les composés organométalliques utilisés pour la préparation du catalyseur sont choisis de préférence dans le groupe constitué par les alkylmétaux, les arylmétaux, les alkylarylmétaux, les aralkylmétaux d'étain, de germanium et/ou de plomb.

La quantité de métal à ajouter est en général comprise entre 0,1 et 20 % en poids (exprimée en élément métal) et avantageusement entre 0,5 et 10 % par rapport à la masse de nickel de Raney.

Les exemples suivants, non limitatifs, illustrent l'invention.

**EXEMPLE 1.**

La préparation du catalyseur se fait en deux étapes:

- fixation du nickel par imprégnation d'acétate de nickel en solution ammoniacale sur une silice dont la surface spécifique est égale à 280 $m^2$ par gramme et le volume poreux total est égal à 80 cm pour 100 grammes, suivie d'une filtration, d'un séchage à 110°C, d'une calcination sous air à 450°C et d'une réduction sous hydrogène à 450°C.
- fixation de l'étain sur le support préimprégné par le nickel, calciné et réduit, sous forme de tétraéthyl-étain en solution dans le normal-heptane. Après avoir laissé en contact pendant 4 heures le catalyseur et la solution de tétraéthylétain au reflux de l'heptane, le catalyseur est lavé par l'heptane puis séché.

Le catalyseur est ensuite chargé dans un réacteur tubulaire puis réduit pendant 4 heures à 300°C sous courant d'hydrogène.

Les conditions opératoires pour l'hydrogénolyse de l'acétate d'ethyle sont les suivantes:

- Pression: 50 bars (5,0 MPa)
- PPH: 4 kg/kg de catalyseur/h
- rapport molaire $H_2$/ester: 5.

Dans cette première serie d'essais on a fait varier la teneur en étain des catalyseurs en partant d'un catalyseur de base contenant 2,5 % en poids de nickel. La température de travail a été fixée à 250°C. Les résultats sont donnés dans le tableau 1.

**TABLEAU 1.**

| % Ni (% pds) | Sn (% pds) | Conversion totale (% pds) | rendement (éthanol) (% pds) |
|---|---|---|---|
| 2,5 | 0 | 42 | 0,9 |
| 2,5 | 0,5 | 3,5 | 2,4 |
| 2,5 | 0,8 | 6,2 | 5,8 |
| 2,5 | 1,5 | 14 | 13,1 |
| 2,5 | 2,3 | 26,2 | 22,9 |
| 2,5 | 2,6 | 32,1 | 31,1 |
| 2,5 | 3,0 | 37,5 | 36,4 |
| 2,5 | 10,0 | 22,0 | 21,9 |

**EXEMPLE 2.**

On compare, à différentes températures, les propriétés catalytiques, vis-à-vis de l'hydrogénolyse de l'acétate d'éthyle, de deux catalyseurs: l'un à base de 2,5 % de nickel sur la silice de l'exemple 1, l'autre à base de 2,5 % de nickel et 3,0 % d'étain sur le même support de silice. Toutes les autres conditions sont identiques à celles de l'exemple 1. Les résultats figurent dans le tableau 2.

**TABLEAU 2.**

| Température (°C) | Catalyseur | | | |
|---|---|---|---|---|
| | 2,5 % Ni/$SiO_2$ | | 2,5 % Ni + 3 % Sn/$SiO_2$ | |
| | Conversion (% pds) | Rendement (éthanol) (% pds) | Conversion (% pds) | Rendement (éthanol) (% pds) |
| 200 | 2,3 | 1,6 | 14,2 | 14,1 |
| 220 | 19 | 5,8 | 23,8 | 23,7 |
| 250 | 42 | 0,9 | 37,5 | 36,4 |
| 280 | 99,8 | 0 | 49,5 | 46,5 |

Dans tout l'intervalle de température considéré, on observe que le catalyseur bimétallique nickel-étain déposé sur silice est incomparablement plus sélectif pour la production d'alcool.

**EXEMPLE 3.**

On propose de fabriquer de l'éthanol à partir d'acétate d'éthyle, à différentes températures à l'aide de deux catalyseurs différents: l'un (A) à base de 2,5 à de nickel et 3,0 % d'étain sur silice préparé suivant la méthode décrite dans l'exemple 1, l'autre (B) à base de 2,5 % de nickel et de 3,0 % d'étain préparé en deux étapes comme suit:

- 1ère étape:

Fixation du nickel-par imprégnation d'acétate de nickel en solution ammoniacale sur une silice dont la surface spécifique est de 280 $m^2xg^{-1}$ et le volume poreux total de 0,8 $cm^3xg^{-1}$, suivie d'une filtration, d'un séchage à 110° C, d'une calcination sous air à 450° C et d'une réduction sous hydrogène à 450° C.

- 2ème étape:

Fixation d'étain sur le support préimprégné de nickel, calciné et réduit sous forme de chlorure stanneux en solution aqueuse en maintenant le pH à la valeur de 0,5 unité pH par ajout d'acide chlorhydrique. Le volume de solution employé est égal au volume poreux de la silice. Après avoir laissé en contact pendant 4 heures la solution du précurseur d'étain et le précurseur de catalyseur obtenu à l'étape 1 à température ambiante, le catalyseur est séché.

Les deux catalyseurs A et B sont ensuite activés sous courant d'hydrogène à 300° C pendant 4 heures, puis testés dans les conditions suivantes:

Pression 50 bars (5,0 MPa)
PPH: 4 kg/kg de catalyseur/h
Rapport molaire $H_2$/ester: 5
Température variable.
Les résultats sont donnés dans le tableau 3.

**TABLEAU 3.**

| Température °C | CATALYSEUR | | | |
|---|---|---|---|---|
| | A | | B | |
| | Conversion % poids | Rendement éthanol % pds | Conversion % poids | Rendement éthanol % pds |
| 200 | 14,2 | 14,1 | 16,5 | 12,7 |
| 220 | 23,8 | 23,7 | 28,2 | 20,7 |
| 250 | 37,5 | 36,4 | 48,4 | 31,3 |
| 280 | 49,5 | 46,5 | 61 | 39,5 |

Dans tout l'intervalle de température considéré le catalyseur préparé à partir du tétraéthyl-étain se révèle plus sélectif que celui préparé à partir du chlorure stanneux.

**EXEMPLE 4.**

On propose de fabriquer de l'éthanol à partir d'acétate d'éthyle dans des conditions identiques à celles adoptées dans l'exemple 1. Le catalyseur est à base de nickel (2,5 %) déposé sur silice de l'exemple 1 et d'un deuxième élément du groupe de l'étain, du germanium et du plomb.

Le germanium et le plomb sont imprégnés en solution hydrocarbonée (normal-heptane) respectivement sous forme de tétraéthyl-germanium et de tétraéthyl-plomb. Le catalyseur à l'étain a été décrit à l'exemple 1.

Les catalyseurs, ainsi préparés, sont mis en oeuvre de la même façon que dans l'exemple 1 (T: 250° C, P: 50 bars, PPH: 4, rapport molaire $H_2$/ester: 5).

Les résultats sont donnés dans le tableau 4.

**TABLEAU 4**

| Ni (% pds) | 2ème métal (%pds) | Conversion (% pds) | Rendement (éthanol) (% pds) |
|---|---|---|---|
| 2,5 | 0 | 42 | 0,9 |
| 2,5 | Sn = 3,0 | 37,5 | 36,4 |
| 2,5 | Ge = 1,8 | 36,9 | 35,7 |
| 2,5 | Pb = 5,2 | 37,1 | 36,0 |

L'étain peut donc être remplacé par le germanium et le plomb sans altération significative des propriétés catalytiques de la masse active.

**EXEMPLE 5.**

On prépare selon la méthode décrite dans l'exemple 1, des catalyseurs à base de nickel et d'étain sur des supports à base d'aluminates de métaux alcalins, alcalino-terreux, de zinc ou de cadmium. Ces supports ont des surfaces spécifiques comprises entre 80 et 150, mètres carré par gramme et des volumes poreux compris entre 50 et 100 $cm^3$ pour 100 grammes. Les pourcentages respectifs de nickel et d'étain sont donnés dans le tableau 5.

L'hydrogénolyse de l'acétate d'éthyle a été effectuée dans des conditions identiques à celles de l'exemple 1. Les résultats sont rassemblés dans le tableau 5.

**TABLEAU 5.**

| Support | % Ni (% pds) | % Sn (% pds) | Conversion (% pds) | Rendement en éthanol (%pds) |
|---|---|---|---|---|
| aluminate de sodium | 2,5 | 3,1 | 35,8 | 34,7 |
| aluminate de potassium | 2,5 | 3,2 | 36,1 | 35 |
| aluminate de magnesium | 2,5 | 3,0 | 35,6 | 34,9 |
| aluminate de calcium | 2,5 | 2,9 | 35,9 | 35,2 |
| aluminate de barium | 2,5 | 3,1 | 36,0 | 35,6 |
| aluminate de zinc | 2,5 | 3,0 | 35,7 | 35,3 |
| aluminate de cadmium | 2,5 | 3,0 | 36,2 | 35,1 |
| aluminate de Mg et Ca | 2,5 | 3,0 | 35,7 | 35,2 |
| aluminate de Ca et Ba | 2,5 | 2,9 | 36,1 | 35,7 |
| aluminate de Zn et Mg | 2,5 | 3,2 | 36,0 | 35,5 |

On peut donc avantageusement utiliser les aluminates de métaux alcalins, alcalino-terreux et/ou de zinc et/ou de cadmium comme supports de catalyseur.

**EXEMPLE 6.**

On se propose de fabriquer différents alcools à partir de divers esters en présence d'un catalyseur à base de nickel (2,5 %) et d'étain (3 %) déposés sur silice et dans des conditions opératoires identiques à celles de l'exemple 1.

Les esters utilisés sont les suivants:
- acétate de butyle secondaire,
- acétate d'amyle,
- acétate d'hexyle,
- caprate d'éthyle,
- palmitate de méthyle,
- oléate de méthyle.

Les résultats sont groupés dans le tableau 6.

**TABLEAU 6.**

| Substrat | Alcools obtenus | Conversion (% pds) | Rendement alcools (% pds) |
|---|---|---|---|
| Acétate de butyle secondaire | Ethanol et butanol-2 | 36,3 | 35,1 |
| Acétate d'amyle | Ethanol et alcool amylique | 34,5 | 33,1 |
| Acétate d'hexyle | Ethanol et hexanol-1 | 36,1 | 34,8 |
| Caprate d'éthyle | Décanol-1 et éthanol | 35,5 | 33,9 |
| Palmitate de méthyle | Hexadécanol-1 et méthanol | 33,1 | 31,7 |
| Oléate de méthyle | Octadécanol-1 et méthanol | 34,2 | 33,0 |

**EXEMPLE 7.**

Dans un réacteur de type Grignard pouvant travailler sous pressions élevées(jusqu'à 80 bars), muni d'un système d'agitation, de systèmes de contrôle et de régulation de la température et de la pression, d'une capacité de 500 ml, on introduit une suspension de nickel de Raney (10 grammes) dans 100 ml d'acetate d'éthyle à température ordinarie, puis en agitant on injecte une solution de tétrabutylétain dans l'acétate d'éthyle en quantité telle que la teneur en étain dans la phase réactionnelle liquide soit égale à 10 % en poids par rapport au poids du nickel de Raney. La suspension est progressivement chauffée sous agitation constante jusqu'à 220°C; lorsque cette température est atteinte on introduit l'hydrogène sous pression de manière à obtenir une pression totale de 50 bars. Après une heure de réaction à 220°C et sous une pression de 50 bars, le produit de réaction est récupéré. La composition de l'effluent de réaction, après détente du réacteur à la pression atmosphérique, est obtenue par analyse des phases gazeuse et liquide. Les principaux constituants sont:

. méthane: 0,2 % en poids
. éthane: 0,6 % en poids
. éthanol: 79 % en poids
. acétate d'éthyle: 20,2 % en poids.

La sélectivité en éthanol est de 99%.

**EXEMPLE 8.** (comparatif)

On réalise la réduction de l'acétate d'éthyle dans le même réacteur que celui employé dans l'exemple 7 et dans les mêmes conditions opératoires à l'exception du fait que l'on n'introduit pas d'étain.

La composition de l'effluent de réaction, après détente du réacteur à la pression atmosphérique, est déterminée par analyse des phases gazeuse et liquide. Les principaux, constituants sont les suivants:

. méthane: 25,3 % en poids
. éthane: 8,5 % en poids
. éthanol: 18 % en poids
. acétate d'éthyle: 48,2 % en poids

La sélectivité en éthanol est de 34,7 %.

## Revendications

1. Procédé de fabrication d'alcools dans lequel un ester d'acide carboxylique est traité par l'hydrogène en présence d'un catalyseur contenant du nickel et au moins un second élément choisi dans le groupe constitué du germanium, de l'étain et du plomb caractérisé en ce que le catalyseur résulte de l'incorporation d'au moins un composé d'au moins un métal du groupe germanium, étain et plomb à du nickel de Raney ou à un support contenant du nickel.

2. Procédé selon la revendication 1 dans lequel le catalyseur renferme sur un support de 0,1 à 60 % en poids de nickel et de 0,1 à 20 % en poids d'au moins un élément du groupe germanium, étain, plomb.

3. Procédé selon la revendication 1 ou 2, dans lequel le rapport atomique entre le ou les éléments du groupe Sn, Ge et Pb présents dans le catalyseur et le nickel est de 0,01:1 à 4:1.

4. Procédé selon la revendication 1 dans lequel le catalyseur renferme du nickel de Raney et, par rapport au nickel de Raney, de 0,1 à 20 % en poids d'au moins un élément du groupe germanium, étain, plomb.

5. Procédé selon l'une des revendications 1 à 3, dans lequel le support présente une surface de 10 à 500 $m^2xg^{-1}$ et un volume poreux de 0,2 à 1,3 $cm^3xg^{-1}$.

6. Procédé selon l'une des revendications 1 à 3 et 5 dans lequel le support contenant du nickel est de la silice.

7. Procédé selon l'une des revendications 1 à 3, et 5 dans lequel le support contenant du nickel est un aluminate d'un élément des groupes $I_A$, $II_A$, ou $II_B$ de la classification périodique des éléments ou un aluminate mixte ou de l'alumine.

8. Procédé selon l'une des revendications 1 à 7 dans lequel le composé d'au moins un métal du groupe germanium, étain, plomb est un hydrocarbylgermanium, un hydrocarbylétain ou un hydrocarbylplomb.

9. Procédé selon l'une des revendications 1 à 8 dans lequel le catalyseur est obtenu par imprégnation d'un support par une solution aqueuse ou organique d'au moins un composé de nickel, séchage du support imprégné suivi d'une calcination à une température de environ 110° C à environ 600° C, puis d'une réduction sous hydrogène à une température de environ 200° C à environ 600° C et enfin introduction d'au moins un composé d'au moins un métal du groupe constitué du germanium, de l'étain et du plomb.

10. Procédé de fabrication d'alcools selon l'une des revendicarions 1 à 9 dans lequel la pression est de 1 à 30 MPa, la température de 180 à 330° C, et le rapport molaire hydrogène/ester est de 2:1 à 50:1.

## Patentansprüche

1. Verfahren zur Herstellung von Alkoholen, wobei ein Carbonsäureester mit Wasserstoff in Gegenwart eines Katalysators, der Nickel und zumindest ein zweites Element aus der Gruppe Germanium, Zinn und Blei enthält, behandelt wird, dadurch gekennzeichnet, daß der Katalysator aus der Einbeziehung von zumindest einer Verbindung von zumindest einem der Metalle der Gruppe Germanium, Zinn und Blei mit Raney-Nickel oder einem nickelhaltigen Träger stammt.

2. Verfahren nach Anspruch 1, dadurch gekennzeichnet, daß der Katalysator auf einem Träger 0,1 bis 60 Gew.-% Nickel und 0,1 bis 20 Gew.-% an zumindest einem Element der Gruppe Germanium, Zinn und Blei aufweist.

3. Verfahren nach Anspruch 1 oder 2, dadurch gekennzeichnet, daß das Atomverhältnis zwischen dem Element oder den Elementen der Gruppe Zinn, Germanium und Blei, die im Katalysator vorliegen, und Nickel, 0,01 : 1 bis 4 : 1 beträgt.

4. Verfahren nach Anspruch 1, dadurch gekennzeichnet, daß der Katalysator Raney-Nickel und, bezogen auf Raney-Nickel, 0,1 bis 20 Gew.-% zumindest ein Element der Gruppe Germanium, Zinn und Blei aufweist.

5. Verfahren nach einem der Ansprüche 1 bis 3, dadurch gekennzeichnet, daß der Träger eine Oberfläche von 10 bis 500 $m^2x\ g^{-1}$ und ein Porenvolumen von 0,2 bis 1,3 $cm^3\ x\ g^{-1}$ aufweist.

6. Verfahren nach einem der Ansprüche 1 bis 3 und 5, dadurch gekennzeichnet, daß der nickelhaltige Träger aus Siliciumdioxid besteht.

7. Verfahren nach einem der Ansprüche 1 bis 3 und 5, dadurch gekennzeichnet, daß der nickelhaltige Träger ein Aluminat eines Elementes der Gruppen $I_A$, $II_A$ oder $II_B$ des periodischen Systems der Elemente oder ein gemischtes Aluminat oder Aluminiumoxid ist.

8. Verfahren nach einem der Ansprüche 1 bis 7, dadurch gekennzeichnet, daß die Verbindung von zumindest einem Metall der Gruppe Germanium, Zinn und Blei ein Germanium-Kohlenwasserstoff, ein Zinn-Kohlenwasserstoff oder ein Blei-Kohlenwasserstoff ist.

9. Verfahren nach einem der Ansprüche 1 bis 8, dadurch gekennzeichnet, daß der Katalysator erhalten wird durch Imprägnieren eines Trägers mit einer wässrigen oder organischen Lösung von zumindest einer Verbindung von Nickel, Trocknen des imprägnierten Trägers und anschließende Calcinierung bei einer Temperatur von etwa 110°C bis etwa 600°C und dann Reduktion unter Wasserstoff bei einer Temperatur von etwa 200°C bis etwa 600°C und schließlich Einführung von zumindest einer Verbindung von zumindest einem Metall der Gruppe Germanium, Zinn und Blei.

10. Verfahren zur Herstellung von Alkoholen nach einem der Ansprüche 1 bis 9, dadurch gekennzeichnet, daß der Druck 1 bis 30 MPa, die Temperatur 180 bis 330°C und das molare Verhältnis Wasserstoff/Ester 2 : 1 bis 50 : 1 betragen.

**Claims**

1 - A process for manufacturing alcohols, wherein a carboxylic acid ester is treated with hydrogen in the presence of a catalyst containing nickel and at least one second element selected from the group consisting of germanium, tin and lead, characterized in that the catalyst results from the incorporation of at least one compound of at least one metal from the germanium, tin and lead group to a Raney nickel or to a nickel-containing carrier.

2 - A process according to claim 1, wherein the catalyst comprises a carrier and from 0.1 to 60 % by weight of nickel and 0.1 to 20 % by weight of at least one element from the germanium, tin and lead group.

3 - A process according to claim 1 or 2, wherein the atomic ratio of the one or more elements from the Sn, Ge and Pb group present in the catalyst to nickel is from 0.01 : 1 to 4 : 1.

4 - A process according to claim 1, wherein the catalyst contains Raney nickel and, in proportion to Raney nickel, from 0.1 to 20 % by weight of at least one element from the germanium, tin and lead group.

5 - A process according to one of claims 1 to 3 wherein the carrier has a surface from 10 to 500 $m^2 \times g^{-1}$ and a pore volume from 0.2 to 1.3 $cm^3 \times g^{-1}$.

6 - A process according to one of claims 1 to 3 and 5, wherein the nickel-containing carrier is silica.

7 - A process according to one of claims 1 to 3 and 5 wherein the nickel-containing carrier is an aluminate of an element from groups $I_A$, $II_A$ or $II_B$ of the periodic classification of elements or a mixed aluminate or alumina.

8 - A process according to one fo claims 1 to 7 wherein the compound of at least one metal from the germanium, tin and lead group is a hydrocarbyl-germanium, a hydrocarbyl-tin or a hydrocarbyl-lead.

9 - A process according to one of claims 1 to 7 wherein the catalyst is obtained by impregnation of a carrier with an aqueous or organic solution of at least one nickel compound, drying of the impregnated carrier followed with roasting at a temperature from about 110°C to about 600°C, then with reduction in hydrogen at a temperature from about 200°C to about 600° and finally with the introduction of at least one compound of at least one metal of the group consisting of germanium, tin and lead.

10 - A process for manufacturing alcohols according to one of claims 1 to 9, wherein the pressure ranges from 1 to 30 MPa, the temperature from 180 to 330°C and the molar ratio hydrogen/ester from 2 : 1 to 50 : 1.